# FASCICULE DE BREVET EUROPEEN

(11) **EP 2 640 290 B1**
(45) Date de publication et mention de la délivrance du brevet: **29.07.2015**
(21) Numéro de dépôt: 11805074.9
(22) Date de dépôt: 16.11.2011
(51) Int. Cl.: A61B 17/88, A61F 2/46

(54) **DISPOSITIF POUR L'INJECTION D'UN CIMENT OSSEUX COMPRENANT UN SYSTEME DE BLOCAGE DE SUR-PRESSION**
VORRICHTUNG ZUR INJEKTION VON KNOCHENZEMENT MIT EINEM ÜBERDRUCKSICHERUNGSSYSTEM
DEVICE FOR THE INJECTION OF BONE CEMENT, COMPRISING AN OVERPRESSURE LOCKING SYSTEM

(30) Priorité: 16.11.2010 FR 1059426
(43) Date de publication de la demande: 25.09.2013
(73) Titulaire: Teknimed, 65502 Vic-en-Bigorre Cedex (FR)
(72) Inventeur: BONNIN, Freddy, F-31500 Toulouse (FR); LEONARD, Alain, F-65500 Caixon (FR)
(74) Mandataire: Hartmann, Jean-Luc
(86) Numéro de dépôt international: PCT/FR2011/052666
(87) Numéro de publication internationale: WO 2012/066238

(56) Documents cités:
- EP-A1- 1 400 213
- US-A- 4 498 904
- US-A1- 2002 013 553
- US-A1- 2003 018 339
- US-A1- 2004 204 715

## Description

La présente invention se rapporte à un dispositif médical pour l'injection de ciment osseux.

L'injection de ciment osseux est réalisée dans diverses opérations afin de consolider la structure de l'os après traumatisme, usure naturelle ou bien encore dans le cas dé maladie dégénérative. Les ciments utilisés au cours de ces opérations sont de composition variable, généralement à base de polymères de méthacrylate associés à d'autres adjuvants selon les applications envisagées. La viscosité de ces compositions est donc elle aussi variable.

### Art antérieur

Divers dispositifs ont été proposés afin de permettre l'injection de ciment osseux au cours de ces opérations. L'injection doit pouvoir être réalisée avec précision et sécurité. L'une des difficultés est d'obtenir un écoulement homogène du ciment osseux à travers la seringue d'injection.

La demande US 2002/013553 décrit un dispositif utilisant une seringue montée sur un manche dont le piston peut être mis en mouvement par l'intermédiaire d'une vis prenant appui sur le manche et transformant les mouvements de rotation effectués par l'opérateur en une translation longitudinale résultant en une progression du piston à l'intérieur de la seringue.

Ce dispositif présente l'avantage de permettre à l'opérateur de réaliser une injection de manière progressive et régulière, le ciment étant évacué précisément à mesure que la pression monte à l'intérieur de la seringue sous l'effet des mouvements de rotation réalisés par l'opérateur.

Cependant, un inconvénient majeur est lié à l'utilisation de ce dispositif dans le cas où l'opérateur effectue un nombre trop important de rotation au niveau de la vis. Ceci génère une surpression à l'intérieur de la seringue entraînant un surplus d'écoulement de ciment dans le meilleur des cas, voire une rupture des parois de la seringue si la surpression est vraiment trop importante.

Il en résulte des conséquences qui peuvent être dramatiques pour la suite de l'opération et donc pour la santé du patient.

D'autres dispositifs ont été décrits dans les demandes US 2004/0204715, US 2003/018339, EP 1400213 ou US 4,498,904, mais les systèmes présentés dans ces demandes ne comprennent pas de mécanismes permettant de réaliser une injection de ciment osseux avec précision, tout en évitant la survenue d'une surpression au niveau du réservoir. Aucun de ces documents ne décrit notamment l'interaction d'un disque mâle venant au contact d'un disque femelle sous l'effet d'une force de compression exercée en partie distale de la vis d'injection de manière à s'autobloquer en fonction de la pression exercée à l'intérieur du réservoir.

### Brève description

La présente invention vise à pallier les inconvénients de l'art antérieur en proposant un nouveau dispositif pour l'injection d'un ciment osseux permettant l'évacuation du ciment de manière progressive et homogène à travers une seringue associée à une vis d'injection, ceci sans risque de générer une surpression à l'intérieur de la seringue. Le dispositif selon la présente invention permet en effet d'éviter d'atteindre un niveau de pression seuil qui peut être déterminé par l'opérateur au moyen d'un système d'entraînement capable de s'auto-bloquer, c'est à dire d'empêcher la progression du piston dans la seringue, en cas de surpression à l'intérieur du réservoir. Le dispositif objet de la présente invention vise à obtenir une injection parfaitement contrôlée en terme de débit et exempte de risques de rupture ou de fissure au niveau du matériel d'injection.

### Description détaillée de l'invention

Plus précisément, le dispositif selon l'invention comprend un réservoir destiné à recevoir ledit ciment osseux, doté d'une première extrémité comprenant un orifice de sortie pour l'injection du ciment et d'une seconde extrémité recevant un piston pouvant effectuer des mouvements de translation longitudinale à l'intérieur du corps du réservoir, ledit piston étant mis en mouvement par l'intermédiaire d'une vis d'injection faisant saillie en dehors du corps du réservoir et étant en prise avec des moyens de préhension aptes à être mis en contact de manière solidaire avec le réservoir, caractérisé en ce qu'il comprend des moyens d'entraînement en rotation de la vis d'injection capables de s'auto-bloquer en fonction de la pression exercée à l'intérieur du corps du réservoir, lesdits moyens d'entraînement en rotation de la vis d'injection comprenant une poignée dotée d'un passage recevant la vis d'injection et s'articulant avec elle par l'intermédiaire d'un couple de disques mâle/femelle, l'un des deux disques, dit disque entraîneur, étant solidaire des mouvements de rotation de la poignée et apte à effectuer des mouvements de translation dans l'axe longitudinal du passage de la poignée, l'autre disque, dit disque entraîné, étant solidaire des mouvements de rotation de la vis d'injection, le disque entraîneur venant au contact du disque entraîné sous l'effet d'une force de compression exercée en partie distale de la vis d'injection.

Dans un mode particulier de réalisation de l'invention, les deux disques entrent en contact l'un avec l'autre au moyen d'un ressort de compression présentant une extrémité en butée au niveau de la partie distale de la vis d'injection, l'autre extrémité du ressort venant au contact, directement ou indirectement, du disque entraîneur. Par exemple, l'autre extrémité du ressort peut venir au contact de la poignée d'injection de telle sorte que celle-ci transmette la force de compression reçue par le ressort au disque entraîneur. Le disque entraîneur est donc mis en contact avec le disque entraîné avec une force plus ou moins élevée en fonction du niveau d'étirement du ressort de compression. Selon le niveau de pression exercée à l'intérieur du réservoir, la force transmise par le ressort de compression est suffisante ou non pour permettre au disque entraîneur de mettre en mouvement le disque entraîné.

De manière avantageuse, la partie distale de la vis d'injection présente des moyens de serrage permettant de régler la distance de compression du ressort. En fonction de la distance choisie, il est ainsi possible d'obtenir une plus ou moins grande sensibilité du dispositif, c'est à dire un blocage des moyens d'entraînement susceptible d'intervenir à des niveaux de pression à l'intérieur du réservoir relativement bas.

En effet, plus la distance de compression est grande, dans la limite d'une compression du ressort a minima, moins la force exercée par le ressort sur le disque entraîneur (ou sur la poignée d'injection) est importante. De ce fait, l'emprise du disque entraîneur sur le disque entraîné ne permet de mettre en rotation la vis d'injection que jusqu'à l'apparition d'un niveau de pression à l'intérieur du réservoir relativement faible. Au-delà de cette pression seuil, la force exercée par le ressort de compression sur le disque entraîneur n'est pas suffisante pour mettre en mouvement le disque entraîné et par incidence la vis d'injection elle-même.

A l'inverse, si la distance de compression est réduite au minimum, c'est à dire de manière à ce que le ressort de compression puisse s'étirer a minima, les mouvements de rotation sont transmis de manière plus directe au disque entraîneur (ou à la poignée d'injection). Celui-ci met en mouvement le disque entraîné avec un effet d'amortissement minimum. Le disque entraîné met donc en rotation la vis d'injection jusqu'à l'apparition de niveaux de pression à l'intérieur du réservoir beaucoup plus élevés.

Il est ainsi possible de choisir précisément la distance de compression du ressort la plus adaptée par simple réglage des moyens de serrage, selon le type d'opération à réaliser, de manière à obtenir une injection de ciment la plus régulière et la plus fine possible en fonction de la viscosité du ciment osseux et des propriétés mécaniques du ressort de compression.

Dans un mode de réalisation particulier, les moyens de préhension du dispositif selon l'invention sont constitués par toute structure dotée d'une forme permettant une prise en main aisée, de préférence dans une seule main de manière à permettre à l'opérateur de se servir de son autre main pour mettre en mouvement la poignée d'injection. De manière avantageuse, les moyens de préhension comprennent un pas de vis complémentaire à celui qui est présent sur la vis d'injection.

Le pas de vis présent sur les moyens de préhension peut être mis en contact avec la vis d'injection par l'intermédiaire d'une pièce d'entraînement. Une telle pièce peut, par exemple, être insérée à l'intérieur des moyens de préhension et dotée de mouvements indépendants par rapport à ceux-ci de manière à permettre l'accouplement ou la désolidarisation du pas de vis des moyens de préhension avec le pas de vis de la vis d'injection. Il est ainsi possible, lorsque les deux pas de vis ne sont pas accouplés, de réaliser des mouvements de translation longitudinale de la vis d'injection sans devoir nécessairement la mettre en rotation, ce qui peut représenter un gain de temps, notamment au moment du chargement du réservoir en ciment osseux.

De manière préférée, la pièce d'entraînement est associée à un ressort de rappel venant en butée sur les moyens de préhension afin de faciliter la mise en contact ou la séparation du pas de vis présent sur les moyens de préhension avec le pas de vis présent sur la vis d'injection. Dans ce cas, avantageusement, les moyens de préhension comprennent une bague de blocage/déblocage associée à une goupille permettant de comprimer le ressort de rappel pour passer de la position séparée à la position de contact entre les deux pas de vis.

### Exemple

Les exemples et figures qui suivent sont donnés à titre purement illustratif et ne doivent pas être interprétés comme une quelconque limitation de la présente invention.
- Figure 1 : vue d'ensemble, de profil et en légère perspective, du dispositif selon l'invention, la vis d'injection étant en position rentrée,
- Figure 2 : vue d'ensemble, de profil et en légère perspective, du dispositif selon l'invention, la vis d'injection étant en position sortie,
- Figure 3 : vue d'ensemble éclatée, de profil et en légère perspective, du dispositif selon l'invention,
- Figure 4 : vue d'ensemble en coupe transversale dans l'axe longitudinal du dispositif selon l'invention, la vis d'injection étant en position rentrée,
- Figure 5 : vue de détail, de profil, des moyens d'entraînement en position non accouplée,
- Figure 6 : vue de détail, en légère perspective, des moyens d'entraînement en position accouplée.

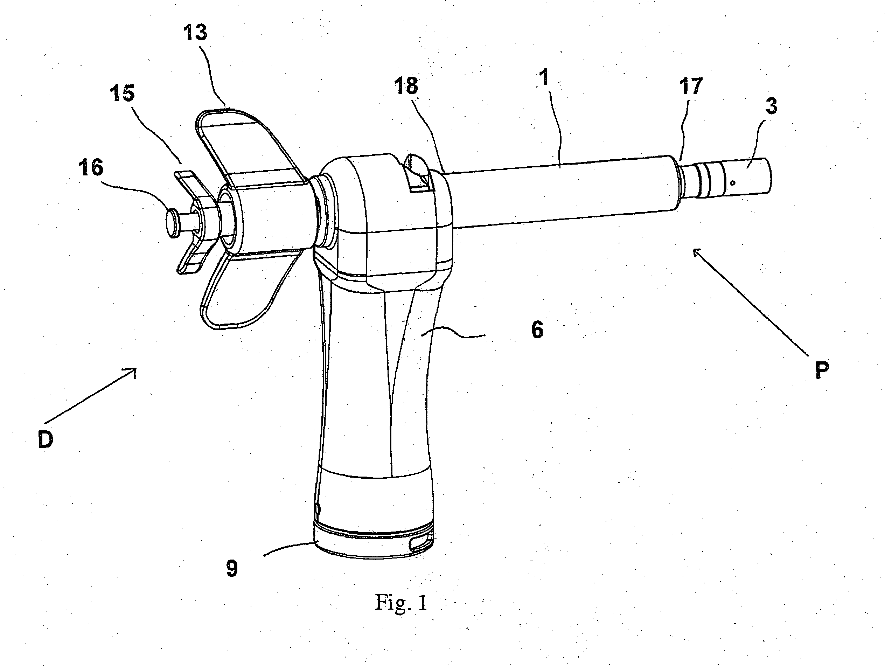

La figure 1 illustre le dispositif d'injection selon l'invention dans son ensemble.

Le réservoir 1 destiné à recevoir le ciment à injecter est doté d'un orifice de sortie au niveau d'une première extrémité 17 capable de recevoir tout connecteur 3 permettant l'acheminement du ciment au niveau du site cible. La seconde extrémité 18 du réservoir 1 est capable de recevoir un piston 2 (non représenté sur la figure 1), mis en mouvement par une vis d'injection 5 (également non représentée). Le réservoir 1 est en prise par son extrémité 18 avec des moyens de préhension 6 ayant la forme d'un manche. Le tout étant situé au niveau de la partie proximale P du dispositif.

La partie distale D du dispositif, située de l'autre côté des moyens de préhension 6, comprend une poignée d'injection 13, articulée avec une molette de serrage 15 et une butée 16.

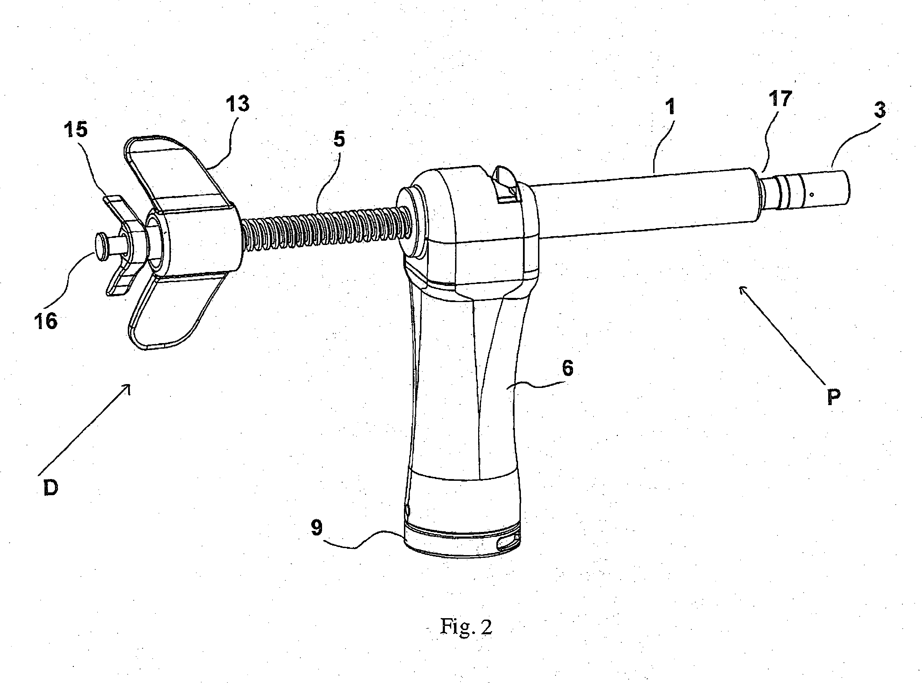

La figure 2 illustre le dispositif selon l'invention en position « chargée » (ou apte à être chargée), c'est à dire avec la vis d'injection 5 en position sortie relativement au corps du réservoir 1. De cette manière, l'opérateur est en mesure d'introduire par aspiration au niveau de la première extrémité 17 le ciment préparé à l'avance à l'intérieur du corps du réservoir 5.

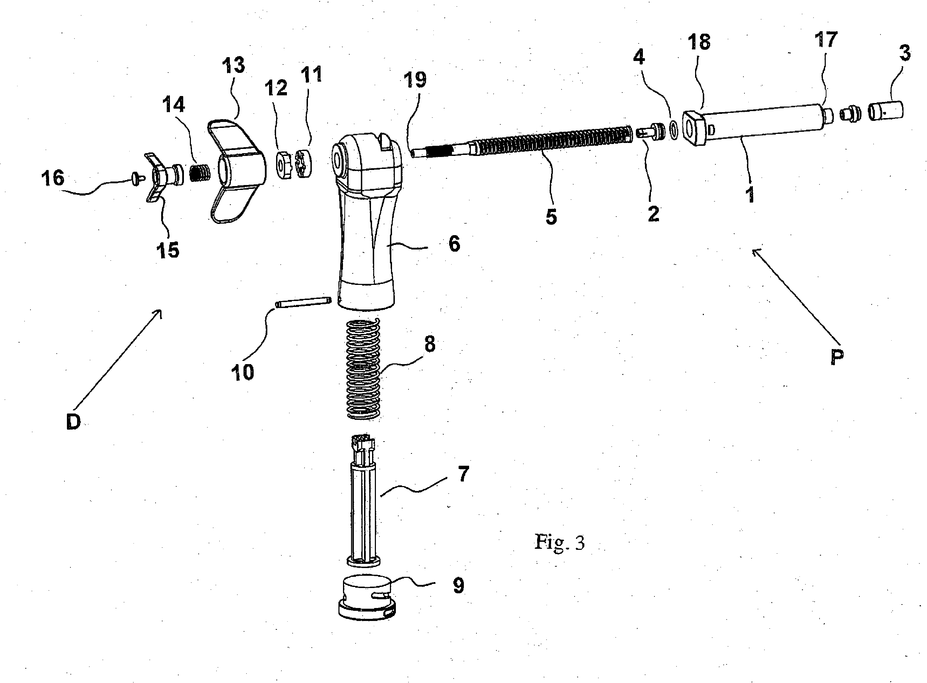

La figure 3 permet d'identifier plus précisément les différents composants du dispositif selon l'invention en vue éclatée. Le réservoir 1 reçoit le piston 2 qui peut être muni d'un joint d'étanchéité 4 afin de permettre une parfaite aspiration et une éjection du ciment au travers de l'extrémité 17. Le piston 2 est en contact de manière solidaire avec la vis d'injection 5. La vis d'injection 5 traverse les moyens de préhension 6 par le biais d'un passage de sorte que l'extrémité 19 de la vis se situe dans la partir distale du dispositif, c'est à dire de l'autre côté des moyens de préhension 6. L'extrémité distale 19 de la vis d'injection reçoit le couple de disques mâle/femelle, la poignée d'injection 13, le ressort de compression 14, les moyens de serrage 15 et la butée 16.

Les moyens de préhension 6 présentent une cavité à l'intérieur de laquelle une pièce d'entraînement 7 peut être logée. Cette dernière est en contact avec un ressort de rappel 8. Les moyens de préhension 6 sont refermés par une bague de blocage/déblocage 9 permettant le maintien de la pièce d'entraînement 7 à l'intérieur de la cavité et capable de recevoir une goupille 10 permettant de maintenir la position bloquée ou débloquée.

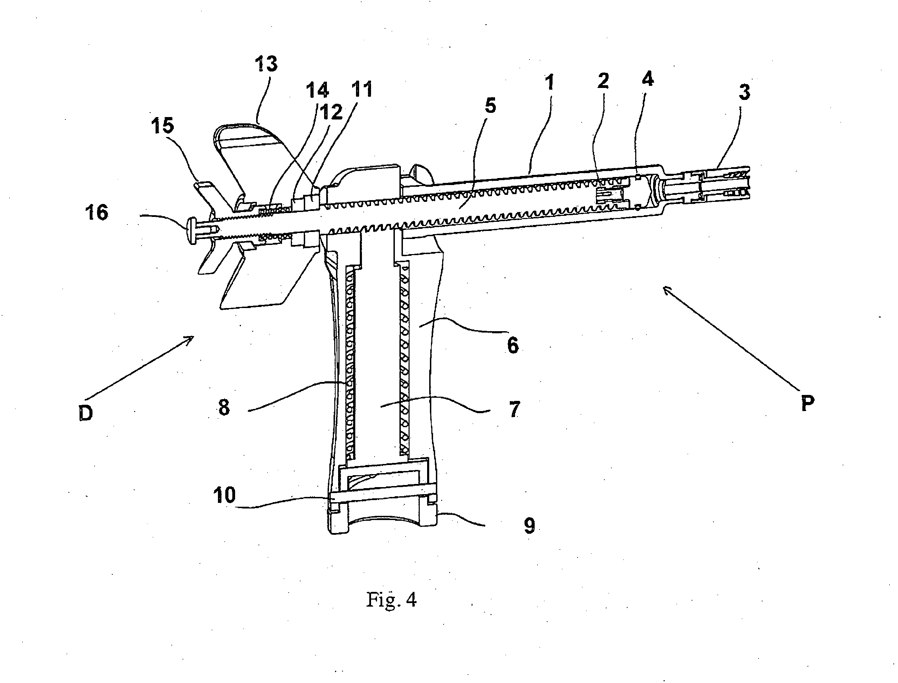

La figure 4 permet d'illustrer l'agencement des différentes pièces composant le dispositif objet de l'invention les unes avec les autres par une représentation en coupe transversale dans l'axe longitudinal. Le dispositif est représenté en position « non chargée », c'est à dire avec la vis d'injection 5 en position rentrée relativement au corps du réservoir 1. Le couple de disques mâle/femelle est accouplé, le disque entraîneur 12 est au contact du disque entraîné 11, lui-même en butée sur une partie de l'extrémité distale 19 de la vis d'injection 5.

Les moyens de préhension 6 sont en position bloquée, le pas de vis présent sur la pièce d'entraînement 7 étant en contact avec le pas de vis de la vis d'injection 5. Cette position est maintenue au moyen de la goupille 10 coopérant avec la bague 9.

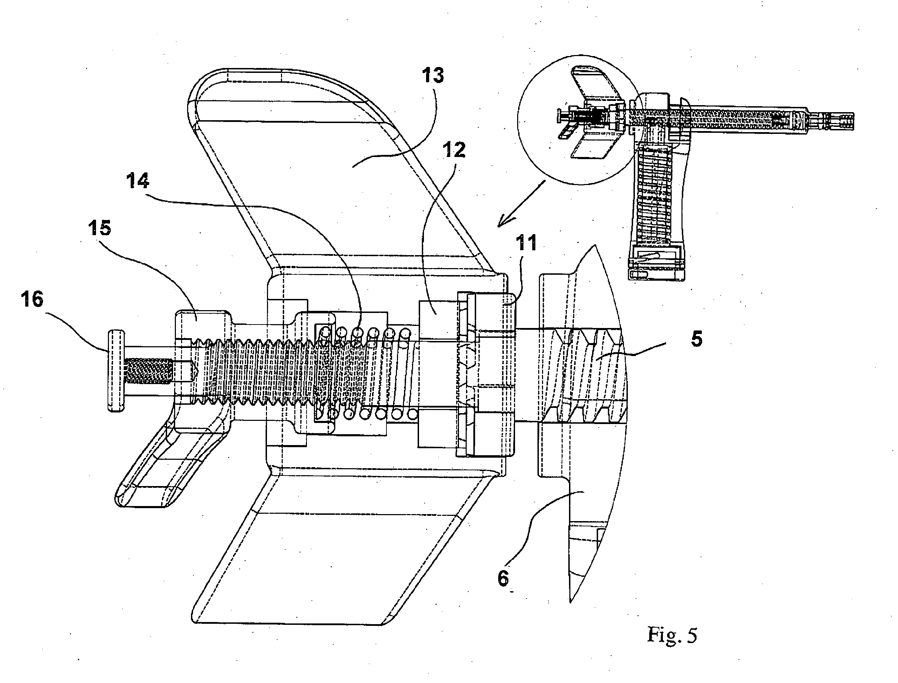

La figure 5 illustre en détail les moyens d'entraînement en position non accouplée. Le disque entraîneur 12 n'est pas en prise avec le disque entraîné 11. La force exercée par le ressort de compression 14 sur le disque entraîneur 12 n'est pas suffisante pour mettre en mouvement le disque entraîné 11.

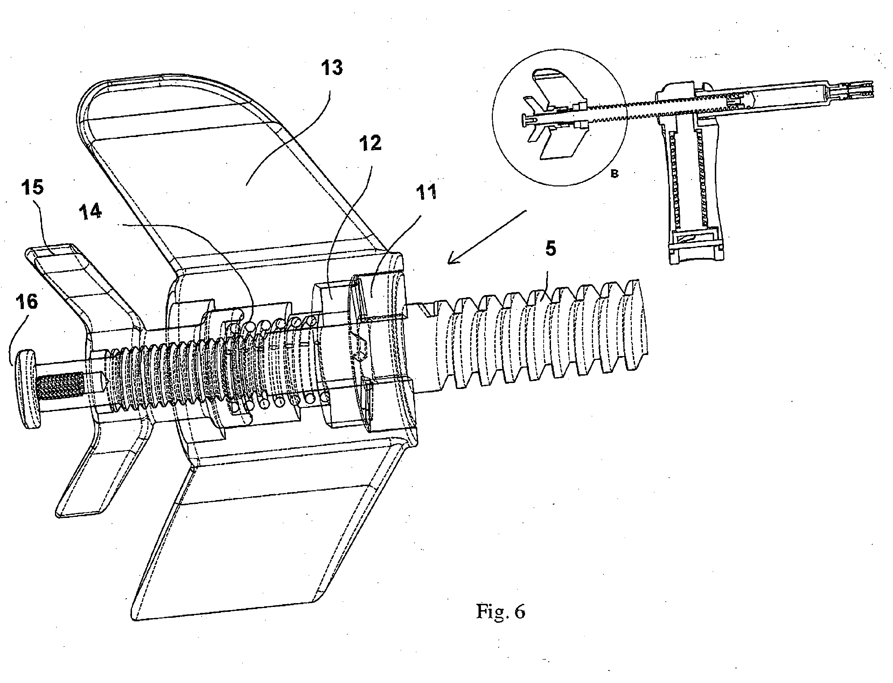

La figure 6 illustre en détail les moyens d'entraînement en position accouplée. Le disque entraîneur 12 est en prise avec le disque entraîné 11. La force exercée par le ressort de compression 14 sur le disque entraîneur 12 permet de mettre en mouvement le disque entraîné 11. Celui-ci entraîne en rotation la vis d'injection 5 qui progresse en translation longitudinale à travers le corps du réservoir 1 via l'accouplement de son pas de vis avec celui de la pièce d'entraînement 7.

### Fonctionnement du dispositif selon l'invention

Au début du procédé d'injection, la pression exercée à l'intérieur du corps du réservoir 1 du dispositif est nulle ou quasi-nulle. Le ressort de compression 14 situé autour de la vis d'injection 5, est en butée par l'une de ses extrémités sur la molette de serrage 15 située à l'extrémité distale 19 de la vis d'injection 5, l'autre extrémité du ressort 14 venant au contact du disque entraîneur 12 solidaire des mouvements de rotation de la poignée d'injection 13 et exerçant ainsi une pression sur celui-ci. La pression initiale exercée en début de procédé par le ressort de compression 14 sur le disque entraîneur 12 provoque la translation longitudinale de celui-ci dans le passage de la poignée 13 et permet de le mettre en contact avec le disque entraîné 11.

La vis d'injection 5 est mise en rotation par l'opérateur en tournant la poignée d'injection 13 dans le sens correspondant à la progression du piston 2 à l'intérieur du corps du réservoir 1 défini par le pas de vis figurant sur les moyens de préhension 6. Le mouvement imprimé à la poignée d'injection 13 est transmis au disque entraîneur 12 qui est lui-même en contact avec le disque entraîné 11 sous l'effet de la pression initiale exercée par le ressort 14. Le disque entraîné 11 suit alors le mouvement de rotation imprimé par le disque entraîneur 12 et provoque la rotation de la vis d'injection 5 dont il est solidaire.

Le mouvement de rotation de la vis d'injection 5 est transformé en une translation dans l'axe longitudinal du dispositif par l'interaction du pas de vis situé sur la vis d'injection 5 avec le pas de vis présent sur les moyens de préhension 6. La vis d'injection 5 progresse alors en translation longitudinale à l'intérieur du réservoir 1 qui est solidaire des moyens de préhension 6 selon les mouvements de rotation que l'opérateur imprime à la poignée d'injection 13. L'extrémité proximale de la vis d'injection 5 est en contact avec un piston 2 permettant de mettre sous pression l'espace du réservoir 1 destiné à recevoir le ciment osseux, encore appelé corps du réservoir.

Plus l'opérateur tourne la poignée d'injection 13, plus la vis d'injection 5 progresse en translation et plus la pression à l'intérieur du réservoir 1 augmente. A partir d'un certain seuil de pression à l'intérieur du réservoir 1, le ciment osseux est ainsi éjecté du réservoir 1 vers l'extérieur via l'orifice de sortie situé à l'extrémité 17. L'écoulement du ciment osseux en dehors du réservoir dépend des propriétés du ciment et notamment de son niveau de viscosité, ainsi que de la pression qui est exercée à l'intérieur du corps du réservoir 1. Plus le ciment est fluide et plus il est facilement évacué du réservoir 1, c'est à dire sous l'effet d'une pression peu élevée. En revanche, plus le ciment est visqueux, plus il est nécessaire d'élever le niveau de pression à l'intérieur du corps du réservoir 1 pour qu'il soit évacué.

Or, plus la pression augmente à l'intérieur du corps du réservoir 1, plus les risques de rupture de paroi de celui-ci sont élevés et moins l'écoulement est régulier et contrôlé. Afin d'éviter que de tels niveaux de pression critique soient atteints et n'entraînent la rupture du réservoir 1 ou un écoulement non contrôlé, la présente invention propose un dispositif doté d'un système d'injection du ciment capable de s'auto-bloquer lorsque la pression à l'intérieur du corps du réservoir 1 dépasse un certain seuil qui peut être pré-déterminé.

Plus la pression exercée à l'intérieur du corps du réservoir 1 est élevée, plus la force nécessaire à la mise en rotation de la vis d'injection 5 est importante. A partir d'un certain niveau de pression défini par les propriétés de résistance mécanique du ressort 14, la force exercée par le ressort 14 sur le disque entraîneur 12 n'est plus suffisante pour permettre à celui-ci de rester en contact avec le disque entraîné 11 de manière à provoquer sa rotation. Le disque entraîneur 12 suit alors les mouvements de rotation transmis par l'opérateur à la poignée d'injection 13, mais il tourne « à vide » sans pouvoir entraîner le disque entraîné 11 solidaire de la vis d'injection 5, laquelle reste bloquée en position sans réaliser de nouvelle rotation et donc sans progresser sur le plan longitudinal.

Lorsque la pression limite, définie par le ressort 14, est atteinte à l'intérieur du corps du réservoir 1, elle diminue progressivement en fonction de la viscosité du ciment à mesure que celui-ci est évacué du réservoir 1 via l'orifice de sortie. On revient ainsi petit à petit à des niveaux de pression qui autorisent la progression par translation longitudinale de la vis d'injection 5 selon des valeurs de force beaucoup moins importantes. Le disque entraîneur 12 peut alors reprendre prise avec le disque entraîné 11 de manière à lui transmettre son propre mouvement de rotation, la force de rotation du disque entraîné 11 redevenant supérieure à la pression exercée à l'intérieur du corps du réservoir 1.

Il est ainsi possible d'injecter progressivement le ciment osseux à l'aide du dispositif selon l'invention sans atteindre des niveaux de surpression à l'intérieur du réservoir 1 susceptibles d'entraîner la rupture de ses parois ou un écoulement irrégulier. L'opérateur peut imprimer des mouvements de rotations à la poignée d'injection 13 et amener la vis d'injection 5 en translation longitudinale jusqu'à atteindre le niveau de pression limite. Quand ce niveau est atteint, l'opérateur peut continuer de tourner la poignée d'injection 13, mais celle-ci n'entraîne plus le disque entraîné 11 solidaire de la vis d'injection 5 et la poignée d'injection 13 tourne alors « à vide » jusqu'à ce que le niveau de pression à l'intérieur du corps du réservoir 1 revienne à des valeurs autorisant l'entraînement du disque entraîné 11 par le disque entraîneur 12.

De manière avantageuse, il est possible de régler la pression limite à partir de laquelle le disque entraîné 11 ne sera plus mis en mouvement par le disque entraîneur 12 en ajustant le niveau de force que le ressort de compression 14 imprime au disque entraîneur 12. Ceci peut être obtenu en sélectionnant la qualité du ressort de compression 14 et le niveau de force qu'il peut transmettre ceci en fonction de sa nature et de sa longueur par exemple. Dans un mode de réalisation particulier de l'invention, le dispositif d'injection comprend des moyens permettant de réduire la longueur de ressort de compression. Une molette de serrage 15 prenant appui sur la vis d'injection 5 peut ainsi être prévue afin de réduire aisément la longueur du ressort de compression 14.

En fonction de la viscosité du ciment à injecter et du niveau de surpression auquel le réservoir 1 peut résister, il est possible de modifier aisément la distance sur laquelle le ressort 14 peut se comprimer et se détendre. Plus cette longueur est réduite, plus le disque entraîneur 12 pourra facilement mettre en rotation le disque entraîné 11, ceci à des niveaux de pression élevés à l'intérieur du corps du réservoir 1. Plus la longueur sur laquelle le ressort 14 peut se détendre est grande et moins le disque entraîneur 12 sera en mesure de mettre en rotation le disque entraîné 11.

## Revendications

1. Dispositif pour l'injection d'un ciment osseux comprenant un réservoir (1) destiné à recevoir ledit ciment osseux, doté d'une première extrémité (17) comprenant un orifice de sortie pour l'injection du ciment et d'une seconde extrémité (18) recevant un piston (2) pouvant effectuer des mouvements de translation longitudinale à l'intérieur du corps du réservoir, ledit piston (2) étant mis en mouvement par l'intermédiaire d'une vis d'injection (5) faisant saillie en dehors du corps du réservoir et étant en prise avec des moyens de préhension (6) aptes à être mis en contact de manière solidaire avec le réservoir (1), ***caractérisé en ce qu***'il comprend des moyens d'entraînement en rotation de la vis d'injection (5) capables de s'auto-bloquer en fonction de la pression exercée à l'intérieur du corps du réservoir, lesdits moyens d'entraînement en rotation de la vis d'injection comprenant une poignée (13) dotée d'un passage recevant la vis d'injection (5) et s'articulant avec elle par l'intermédiaire d'un couple de disques mâle/femelle, l'un des deux disques, dit disque entraîneur (12), étant solidaire des mouvements de rotation de la poignée (13) et apte à effectuer des mouvements de translation dans l'axe longitudinal du passage de la poignée, l'autre disque, dit disque entraîné (11), étant solidaire des mouvements de rotation de la vis d'injection (5), le disque entraîneur (12) venant au contact du disque entraîné (11) sous l'effet d'une force de compression exercée en partie distale (19) de la vis d'injection (5).

2. Dispositif pour l'injection d'un ciment osseux selon la revendication 1, ***caractérisé en ce que*** les deux disques (11,12) entrent en contact l'un avec l'autre au moyen d'un ressort de compression (14) présentant une extrémité en butée au niveau de la partie distale (19) de la vis d'injection, l'autre extrémité du ressort venant au contact, directement ou indirectement, du disque entraîneur (12).

3. Dispositif pour l'injection d'un ciment osseux selon la revendication 2, ***caractérisé en ce que*** la partie distale (19) de la vis d'injection (5) présente des moyens de serrage (15) permettant de régler la distance de compression du ressort.

4. Dispositif pour l'injection d'un ciment osseux selon l'une quelconque des revendications 1 à 3, ***caractérisé en ce que*** les moyens de préhension (6) comprennent un pas de vis complémentaire à celui qui est présent sur la vis d'injection (5).

5. Dispositif pour l'injection d'un ciment osseux selon la revendication 4, ***caractérisé en ce que*** le pas de vis présent sur les moyens de préhension (6) est mis en contact avec la vis d'injection (5) par l'intermédiaire d'une pièce d'entraînement (7).

6. Dispositif pour l'injection d'un ciment osseux selon la revendication 5, ***caractérisé en ce que*** la pièce d'entraînement (7) est associée à un ressort de rappel (8) venant en butée sur les moyens de préhension (6).

7. Dispositif pour l'injection d'un ciment osseux selon la revendication 6, ***caractérisé en ce que*** les moyens de préhension (6) comprennent une bague de blocage/déblocage (9) associée à une goupille (10) permettant de comprimer le ressort de rappel (8).

## Patentansprüche

1. Vorrichtung zum Einspritzen eines Knochenzements mit einem Behälter, der dazu bestimmt ist, diesen Knochenzement aufzunehmen, und mit einem ersten Ende (17), das eine Ausgangsöffnung zum Einspritzen des Zements enthält und mit einem zweiten Ende (18) versehen ist, das einen Kolben (2) aufnimmt, der Längstranslationsbewegungen ins Körperinnere des Behälters ausführen kann, wobei der Kolben (2) mittels einer Einspritzschnecke (5) in Bewegung gesetzt wird, die nach außen vom Körper des Behälters übersteht und die sich mit Greifmitteln im Eingriff befinden, die geeignet sind, mit dem Behälter in festen Kontakt gebracht zu werden,
**dadurch gekennzeichnet, dass**
sie Drehantriebsmittel der Einspritzschnecke (5) enthält, die fähig sind, sich aufgrund eines im Inneren des Behälterkörpers ausgeübten Drucks selbst zu blockieren, wobei diese Drehantriebsmittel der Einspritzschraube einen Griff (13) aufweisen, der mit einem die Einspritzschraube (5) aufnehmenden Durchgang ausgestattet ist und mit ihr über eine männliche/weibliche Drehmomentscheibe verbunden ist, wobei eine dieser zwei Scheiben, die als Antriebsscheibe (12) bezeichnet wird, mit den Drehbewegungen des Griffs (13) fest verbunden ist und geeignet ist, die Translationsbewegungen in der Längsachse des Durchgangs des Griffs zu bewirken und wobei die andere Scheibe, die als angetriebene Scheibe (11) bezeichnet wird, mit den Drehbewegungen der Einspritzschnecke (5) fest verbunden ist, wobei die Antriebsscheibe (12) mit der angetriebenen Scheibe (11) durch die Wirkung einer im distalen Bestandteil (19) der Einspritzschraube (5) ausgeübten Kompressionskraft in Kontakt kommt.

2. Vorrichtung zum Einspritzen eines Knochenzements nach Anspruch 1,
**dadurch gekennzeichnet, dass**
die zwei Scheiben (11, 12) miteinander in Kontakt kommen mittels einer Kompressionsfeder (14), die ein Stützende auf Höhe des distalen Bestandteils (19) der Einspritzschraube aufweist, wobei das andere Federende direkt oder indirekt mit der Antriebsscheibe (12) in Kontakt kommt.

3. Vorrichtung zum Einspritzen eines Knochenzements nach Anspruch 2,
**dadurch gekennzeichnet, dass**
der distale Bestandteil (19) der Einspritzschnecke (5) Spannmittel (15) aufweist, die das Regeln des Kompressionshubs der Feder erlauben.

4. Vorrichtung zum Einspritzen eines Knochenzements nach einem der Ansprüche 1 bis 3,
**dadurch gekennzeichnet, dass**
die Greifmittel (6) eine Gewindesteigung enthalten, die zu derjenigen der Einspritzschnecke komplementär ist.

5. Vorrichtung zum Einspritzen eines Knochenzements nach Anspruch 4,
**dadurch gekennzeichnet, dass**
die Gewindesteigung auf den Greifmitteln (6) in Kontakt gebracht wird mit der Einspritzschnecke (5) mittels eines Antriebsstücks (7).

6. Vorrichtung zum Einspritzen eines Knochenzements nach Anspruch 5,
**dadurch gekennzeichnet, dass**
das Getriebestück (7) mit einer Rückstellfeder (8) verbunden ist, die auf den Greifmitteln (6) in Anschlag kommt.

7. Vorrichtung zum Einspritzen eines Knochenzements nach Anspruch 6,
**dadurch gekennzeichnet, dass**
die Greifmittel (6) einen mit einem Stift (10) verbundenen Blockierungs- / Entriegelungsring (9) enthalten, der der Rückstellfeder (8) das Zusammenpressen gestattet.

## Claims

1. Device for the injection of a bone cement, comprising a container (1) intended to receive the said bone cement, provided with a first end (17) including an outlet for the injection of the cement and a second end (18) that receives a piston (2), capable of carrying out longitudinal translation movements in the interior of the body of the container, said piston (2) being set in motion by means of an injection screw (5) that projects from the container body and engages with gripping means (6) capable of being brought into contact in an integral manner with the container (1), ***characterised in that*** it comprises injection screw (5) rotation means that can self-lock depending on the pressure exerted inside the container body, said injection screw rotation means comprising a handle (13) which is provided with a passage that receives the injection screw (5) and which is hinged thereto by means of a pair of male/female disks, one of the two disks, known as the drive disk (12), moving integrally with the rotation movements of the handle (13) and capable of carrying out translation movements in the longitudinal axis of the passage of the handle, and the other disk, known as the driven disk (11), moving integrally with the rotation movements of the injection screw (5), the drive disk (12) coming in contact with the driven disk (11) in response to a compressive force exerted on the distal part (19) of the injection screw (5).

2. Device for the injection of a bone cement according to claim 1, ***characterised in that*** the two disks (11, 12) come into contact with one another by means of a compression spring (14), with one end comprising a stop in the region of the distal part (19) of the injection screw, and the other end of the spring coming in contact, directly or indirectly, with the drive disk (12).

3. Device for the injection of a bone cement according to claim 2, ***characterised in that*** the distal part (19) of the injection screw (5) comprises locking means (15) allowing for the distance of compression of the spring to be adjusted.

4. Device for the injection of a bone cement according to any one of claims 1 to 3, ***characterised in that*** the gripping means (6) comprise a screw thread complementary to that which is present on the injection screw (5).

5. Device for the injection of a bone cement according to claim 4, ***characterised in that*** the screw thread present on the gripping means (6) is brought in contact with the injection screw (5) by means of a drive element (7).

6. Device for the injection of a bone cement according to claim 5, ***characterised in that*** the drive element (7) is associated with a return spring (8) which comes to a stop position on the gripping means (6).

7. Device for the injection of a bone cement according to claim 6, ***characterised in that*** the gripping means (6) comprise a blocking/unblocking bushing (9) associated with a pin (10) which allows the return spring (8) to be compressed.
